# EUROPÄISCHE PATENTSCHRIFT

(11) **EP 4 525 797 B1**
(45) Veröffentlichungstag und Bekanntmachung des Hinweises auf die Patenterteilung: **16.07.2025**
(21) Anmeldenummer: 22761082.1
(22) Anmeldetag: 29.07.2022
(51) Int. Cl.: A61F 13/15, A61F 13/471, A61F 15/00, A47K 10/16, A47K 10/32

(54) **HYGIENEPRODUKT UND SPENDER FÜR HYGIENEPRODUKT**
HYGIENE PRODUCT AND DISPENSER FOR HYGIENE PRODUCT
PRODUIT D'HYGIÈNE ET DISTRIBUTEUR POUR PRODUIT D'HYGIÈNE

(43) Veröffentlichungstag der Anmeldung: 26.03.2025
(73) Patentinhaber: ARSIMO HOLDING GMBH, 49143 Bissendorf (DE)
(72) Erfinder: REHME-SCHLÜTER, Arne, 49143 Bissendorf (DE); DIEKBREDER, Michael, 49074 Osnabrück (DE)
(74) Vertreter: Dr. Träger & Strautmann PAe PartG mbB
(86) Internationale Anmeldenummer: PCT/EP2022/071388
(87) Internationale Veröffentlichungsnummer: WO 2024/022593

(56) Entgegenhaltungen:
- US-A1- 2006 090 250
- US-B1- 6 613 954

## Beschreibung

Die Erfindung betrifft ein Hygieneprodukt zur Reinigung des Intimbereichs gemäß dem Oberbegriff des Anspruchs 1. Des Weiteren betrifft die Erfindung einen Spender zur Abgabe eines derartigen Hygieneprodukts.

In unmittelbarer Umgebung üblicher Toiletten in der Regel Toilettenpapier vorhanden. Dies dient dazu, dass eine Person nach Benutzung der Toilette ihren Intimbereich durch Abwischen damit reinigen kann. Nach Benutzung wird das Toilettenpapier in die Toilette geworfen, um es dann durch den Abfluss wegzuspülen und somit zu entsorgen.

Bei derartigem Toilettenpapier handelt es sich typischerweise um sogenanntes Tissuepapier. Es ist in der Regel ein mehrlagiges zellstoffbasiertes Material. Dieses Material ist üblicherweise einerseits hinreichend reißfest zum Abwischen von Resten des Stuhlgangs und andererseits auch saugfähig zur Aufnahme von Restmengen an Urin. Aufgrund des großen Querschnitts üblicher Toilettenabflüsse zur Aufnahme ist ein Wegspülen des Papiers ohne weiteres möglich. Toilettenpapier lässt sich als zellstoffbasiertes Material in Kläranlagen unter anderem biologischen Reinigungsprozessen zuführen.

Reine Stehtoiletten, sogenannten Urinale, werden üblicherweise von Männern benutzt. Urinale dienen lediglich zur Abgabe von Urin, also zum sogenannten Wasserlassen. Auch in diesem Fall wäre eine Reinigung des Intimbereichs von Restmengen an Urin wünschenswert, damit die Kleidung, insbesondere die Unterwäsche, nicht verschmutzt wird.

Aus der US 6,613,954 B1 ist ein dispergierbares, absorbierendes Produkt auf Basis einer Faserstruktur bekannt. Die US 2006/0090250 A1 beschreibt ein lösliches Toilettenpapier als Urinierhilfe mit einem bahnförmigen Aufbau zur Flüssigkeitsableitung.

Da Urinale aber lediglich zur Aufnahme von Urin vorgesehen sind, weisen deren Abflüsse im Allgemeinen einen lediglich sehr kleinen Querschnitt auf. Häufig sind sogar siebartige Einsätze vorgesehen. Übliches Toilettenpapier wird zwar bekanntlich bei Wasserkontakt weicher und anschmiegsamer, bleibt aber im Wesentlichen in seiner blattförmigen Struktur erhalten. Dies liegt daran, dass die oben bereits beschriebenen Eigenschaften Reißfestigkeit und Saugfähigkeit gleichzeitig gegeben sein müssen. Bei einer Benutzung bekannter Toilettenpapiere verstopfen daher Urinale in der Regel unmittelbar. Dementsprechend ist Toilettenpapier nicht zur Verwendung am und Entsorgung im Urinal geeignet.

Es ist daher eine Aufgabe der Erfindung, diese Nachteile zu überwinden. Insbesondere soll eine einfache Möglichkeit gefunden werden, eine Reinigung des Intimbereichs auch bei einer Benutzung eines Urinals vorzusehen. Eine Verschmutzung der Bekleidung soll vermieden werden. Mikroorganismen sollen aus dem Intimbereich zu Hygienezwecken entfernt werden.

Diese Aufgabe wird gelöst durch das Hygieneprodukt beziehungsweise Hygieneartikel mit den Merkmalen des Anspruchs 1. Es handelt sich um ein Hygieneprodukt, insbesondere für Männer, zur Reinigung des Intimbereichs nach dem Urinieren an einer Toilette, insbesondere einem Urinal. Das Hygieneprodukt ist mit oder aus einem Material zur Aufnahme von Flüssigkeit gebildet, insbesondere einem saugfähigen Material. Das Hygieneprodukt ist insbesondere vollständig aus dem Material Polyvinylalkohol (PVA) gebildet und vollständig wasserlöslich. Das Material ist insbesondere zur Aufnahme von Urinresten, insbesondere Urintropfen, vorgesehen. Insbesondere ist ein Wegspülen des Materials in der Toilette vorgesehen. Das Hygieneprodukt zeichnet sich dadurch aus, dass das Material wasserlöslich ist. Das Hygieneprodukt ist damit in Wasser auflösbar. Auf diese Weise kann das Hygieneprodukt in der Toilette, insbesondere dem Urinal, entsorgt werden. Urin bestehet typischerweise überwiegend aus Wasser. Insbesondere ist daher auch der Urin bereits in der Lage, das Hygieneprodukt beziehungsweise dessen Material zumindest teilweise aufzulösen. Das Hygieneprodukt wird dabei vorzugsweise in der Regel nicht vollständig aufgelöst, da typischerweise lediglich kleine Urinmengen vorhanden sind. Somit kann es dabei insbesondere lediglich angelöst werden. Höhere Temperaturen des Wassers sorgen insbesondere für eine bessere und/oder schnelle Löslichkeit.

Das Material des Hygieneprodukts ist vollständig wasserlöslich. Damit ist das Hygieneprodukt beziehungsweise dessen Material auf einfache Weise zu entsorgen. Es ist dann insbesondere nicht erforderlich, separate Mülleimer zur Entsorgung benutzter Hygieneprodukte am Urinal aufzustellen. Das Material und damit das Hygieneprodukt lässt sich in Wasser auflösen.

Das Material ist insbesondere in kurzer Zeit wasserlöslich. Mit anderen Worten ist das Material und damit das Hygieneprodukt in ausreichend kurzer Zeit löslich, um eine wasserbasierte Entsorgung zu ermöglichen. Dies bedeutet insbesondere, dass es in weniger als fünf Minuten, vorzugsweise in weniger als einer Minute, weiter vorzugsweise weniger als 30 Sekunden wasserlöslich ist. Damit wird erreicht, dass das Hygieneprodukt unmittelbar nach der Benutzung mittels Wasser zu entsorgen ist.

Bevorzugt ist das Material zumindest durch das Wasser der Spülung der Toilette auflösbar und/oder wegspülbar. Vorzugsweise läuft das Spülwasser mit dem aufgelösten Material ab, vorzugsweise durch den Abfluss der Toilette. Folglich ist das Hygieneprodukt nach der Benutzung direkt im Urinal zu entsorgen.

Das Hygieneprodukt beziehungsweise dessen Material ist insbesondere während weniger Spülvorgänge wasserlöslich und/oder wegspülbar. Vorzugsweise ist es zumindest im Wesentlichen innerhalb von zwei Spülvorgängen, weiter vorzugsweise innerhalb eines Spülvorgangs wasserlöslich und/oder wegspülbar. Damit kann eine zeitnahe Beseitigung im Urinal sichergestellt werden. Auf diese Weise können für den nächsten Benutzer störende Reste im Urinal vermieden werden.

Bei dem Hygieneprodukt beziehungsweise dessen Material handelt es sich vorzugsweise um ein textiles Material. Vorzugsweise ist das Material ein nicht-verwobenes Material, insbesondere ein Vlies beziehungsweise Vliesstoff. Insbesondere kann es sich alternativ auch um ein verwobenes Material handeln, insbesondere ein Gewebe. Damit eignet es sich insbesondere als Hygieneprodukt. Im Bereich von zwischen 40 und 60 Gramm pro Quadratmeter ist allgemein insbesondere bereits eine gute Verwendbarkeit von Vlies gegeben. Vorzugsweise wird ein Vlies mit einem Flächengewicht von wenigstens 30 bis 35 Gramm pro Quadratmeter verwendet. Als optimal haben sich insbesondere Flächengewichte von etwa 50 Gramm pro Quadratmeter gezeigt.

Das Material ist vorzugsweise ein biologisch kompatibles Material. Vorzugsweise handelt es sich um ein biologisch abbaubares Material, vorzugsweise ein kompostierbares Material. Somit wird eine nachhaltige Entsorgung möglich. Auch die Ausgangsmaterialien können dann typischerweise aus nachhaltigen Quellen sein.

Das Hygieneprodukt beziehungsweise dessen Material ist insbesondere ein Kunststoff, insbesondere ein Bio-Kunststoff. Es handelt sich um einfach und kostengünstig herstellbare Materialien. Derartige Materialien eignen sich besonders für den Einsatz als erfindungsgemäßes Hygieneprodukt.

Bei dem Material handelt es sich um Polyvinylalkohol (PVA). PVA hat sich als besonders geeignetes Material angeboten. Es ist in geeigneter Form herstellbar. Es lassen sich insbesondere textile Materialien herstellen, wie insbesondere Vliese. Insbesondere lassen sich damit hier gewünschte Eigenschaften, wie beispielsweise Wasserlöslichkeit und Biokompatibilität, aber ebenso auch geeignete Portionsgrößen für die Verwendung als Hygieneartikel erreichen. Die Löslichkeit von PVA in Wasser ist grundsätzlich materialabhängig einstellbar. Dabei lassen sich unterschiedliche Löslichkeiten bei verschiedenen Temperaturen vorsehen. Für die Anwendung hat sich insbesondere bei den bekannten Materialien eine Löslichkeit ab nicht mehr als etwa 25 Grad Celsius als geeignet gezeigt. Idealerweise ist eine Löslichkeit bei niedrigeren Temperaturen vorteilhaft. Auf diese Weise kann auch kaltes Leitungswasser bevorzugt eine schnelle Auflösung sicherstellen. Somit werden Temperaturen, insbesondere als sogenannte Kaltwasserlöslichkeit, von nicht mehr als 30 Grad Celsius bevorzugt, weiter bevorzugt von nicht mehr als 25 Grad Celsius, idealerweise von nicht mehr als 20 Grad Celsius oder 15 Grad Celsius.

Das Material ist insbesondere zur portionsweisen Verwendung vorgesehen. Es sind vorzugsweise Portionen des Materials vordefiniert. Alternativ oder weiter vorzugsweise ist das Material zur Benutzung in Portionen zerteilbar. Damit eignet es sich zur Reinigung des Intimbereichs insbesondere bei kleinen Mengen Urin am Urinal. Es lassen sich damit Verschmutzungen verhindern und/oder unerwünschte Mikroorganismen beseitigen.

Die oben geschilderte Aufgabe wird außerdem gelöst durch einen Spender zur Abgabe eines Hygieneprodukts, insbesondre für Männer, nach obigen Beschreibungen. Der Spender ist besonders zur Anordnung in der Nähe einer Toilette, insbesondere eines Urinals, geeignet. Es ist eine Abgabe des Materials des Hygieneprodukts an eine Person vorgesehen. Das Material zeichnet sich dadurch aus, dass es portionsweise abgebbar ist. Dazu ist der Spender vorgesehen. Das Material ist in kleine Portionen zu entnehmen.

Bevorzugt ist das Hygieneprodukt beziehungsweise das Material durch eine Person entnehmbar. Der Spender ist dazu ausgebildet, eine Entnahme durch eine Person zu ermöglichen. Die Person kann das Material aus dem Spender herausziehen, vorzugsweise in Form einzelner Hygieneartikel. Es lassen sich insbesondere flache, vorzugsweise lappenartige und/oder tuchartige Formen des Hygieneprodukts verwenden. Die Hygieneprodukte weisen insbesondere eine geeignete Größe zur Reinigung auf, vergleichbar mit üblichem Toilettenpapier. Bevorzugt eignen sich Flächen des Hygieneprodukts mit etwa 5 bis 20 Zentimetern Kantenlänge besonders gut, weiter bevorzugt etwa 8 bis 12 Zentimeter Kantenlänge. Damit kommen Flächen der einzelnen Hygieneprodukte von etwa 20 bis 200 Quadratzentimeter in Betracht, vorzugsweise in etwa 80 bis 160 Quadratzentimeter. Weiter bevorzugt sind aber auch tupferartige und/oder padartige und/oder wattebauschartige Formen einsetzbar. Insbesondere sind in allen Fällen rechteckige, runde oder auch beliebige andere freie Formen denkbar.

Vorzugsweise erfolgt bei der Entnahme des Hygieneprodukts beziehungsweise des Materials eine Portionierung. Das Material ist dadurch in Portionen benutzbar. Besonders bevorzugt erfolgt die Portionierung durch die Person. Mit anderen Worten kann das Material des Hygieneprodukts bei der Entnahme aus dem Spender unmittelbar portioniert werden. Bei der Entnahme erfolgt eine Portionierung. Hierzu kann das Material insbesondere vorportioniert sein. Mit anderen Worten können beispielsweise größere Mengen des Materials bevorratet werden, wobei insbesondere Trennstellen vorgegeben sein können. An derartigen Trennstellen lassen sich Portionen des Materials abteilen.

Bei dem Spender ist vorzugsweise eine automatische Abgabe und/oder eine automatische Portionierung des Hygieneprodukts beziehungsweise des Materials vorgesehen. Weiter vorzugsweise ist eine motorische Abgabe und/oder Portionierung vorgesehen. Der Spender bietet durch eine automatische Abgabe beziehungsweise Portionierung eine Abgabe des Hygieneprodukts in geeigneten Mengen beziehungsweise Größen. Damit kann eine passende Menge des Materials abgegeben werden. Alternativ oder zusätzlich kann eine manuelle Entnahme von Hygieneprodukten aus dem Spender durch eine Person vorgesehen sein, vorzugsweise eine vollständig manuelle Entnahme.

Ein bevorzugtes Ausführungsbeispiel der Erfindung wird im Folgenden anhand der Figuren der Zeichnung näher beschrieben. In dieser zeigt:

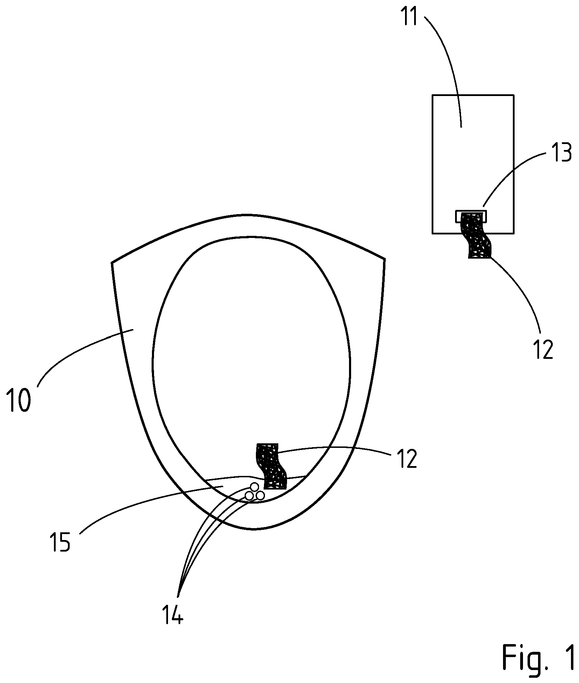

Fig. 1 ein Urinal mit einem Spender zur Abgabe erfindungsgemäßer Hygieneprodukte.

In der Fig. 1 ist als Beispiel für eine Toilette ein Urinal 10 dargestellt. Neben dem Urinal 10 ist ein Spender 11 angeordnet.

Der Spender 11 dient insbesondere dazu, erfindungsgemäße Hygieneprodukte 12 abzugeben.

Zur Abgabe der Hygieneprodukte 12 weist der Spender 11 eine Abgabeöffnung 13 auf. Aus dieser Abgabeöffnung 13 lassen sich einzelne Hygieneprodukte 12 entnehmen.

Im Innern des Spenders 11 ist dazu eine Menge an Material beziehungsweise entsprechender Hygieneartikel 12 vorhanden. Mit anderen Worten ist im Spender 11 ein Vorrat vorgesehen.

Gegebenenfalls kann der Spender 11 zusätzlich eine Vorrichtung enthalten, die eine automatische Abgabe ermöglicht. Dazu kann insbesondere eine motorische Abgabe vorgesehen sein, beispielsweise in Form einer speziellen Abgabevorrichtung.

Nach dem Urinieren an einem Urinal sollen Restmengen an Urin durch die das Urinal benutzende Person beseitigt werden. Hierzu sollen die entsprechenden Hygieneprodukte 12 dienen. Die Person kann ein Hygieneprodukt 12 verwenden, um den Intimbereich zu reinigen. Dazu entnimmt die Person ein Hygieneprodukt 12 aus dem Spender 11 und nutzt es zur Reinigung.

Nach der Benutzung lassen sich die Hygieneprodukte 12 in das Urinal 10 werfen. Dort wird Wasser 15 zur Spülung verwendet. Das Wasser 15 der Toilettenspülung dient zunächst dazu, das Urinal von Resten des Urins zu reinigen. Das Wasser 15 läuft durch Ablauföffnungen 14 im Bodenbereich des Beckens des Urinals 10 ab. Es läuft in den Abfluss hinein, typischerweise weiter in die Kanalisation.

Darüber hinaus wird aber auch das Material des Hygieneprodukts 12 durch das Wasser 15 benetzt. Das Hygieneprodukt 12 ist erfindungsgemäß aus wasserlöslichem Material. Dies führt im Ergebnis dazu, dass das Hygieneprodukt 12 bei Wasserkontakt aufgelöst wird.

Auch der Urin ist bereits in der Lage, das Hygieneprodukt 12 bereits teilweise aufzulösen. Da es sich beim Urin in der Regel um kleine Mengen handelt, wird das Hygieneprodukt 12 dabei typischerweise nicht vollständig aufgelöst. Vielmehr wird es damit angelöst. Bei höheren Temperaturen des Wassers ergibt sich typischerweise eine bessere Lösbarkeit des Materials.

Als Material eignet sich insbesondere Polyvinylalkohol (PVA). Es lässt sich in geeigneter wasserlöslicher Form herstellen. Einerseits kann es Urin bei Reinigung des Intimbereichs aufnehmen. Andererseits kann es gleichzeitig in größeren Mengen Wasser 15 wasserlöslich sein, beispielsweise bei Kontakt mit dem Wasser 15 der Toilettenspülung der Urinals 10.

Bei der Spülung im Urinal 10 ist hinreichend Wasser 15 vorhanden, um das Hygieneprodukt 12 aufzulösen. Das aufgelöste Hygieneprodukt 12 kann dann einfach zusammen mit dem Wasser 15 ebenfalls durch die Ablauföffnungen 14 in den Abfluss fließen.

Jedes einzelne Hygieneprodukt 12 kann dabei zur Reinigung des Intimbereichs verwendet werden. Die Hygieneprodukte 12 können dazu einzeln aus dem Spender 11 entnommen werden. Die Portionierung kann bereits im Material vorgegeben sein oder direkt bei der Entnahme durch die Person erfolgen.

### Bezugszeichenliste

- 10: Urinal
- 11: Spender
- 12: Hygieneprodukt
- 13: Abgabeöffnung
- 14: Abflussöffnung
- 15: Wasser

## Patentansprüche

1. Hygieneprodukt, insbesondere für Männer, zur Reinigung des Intimbereichs nach dem Urinieren an einer Toilette, insbesondere einem Urinal (10), gebildet aus einem Material zur Aufnahme von Flüssigkeit, insbesondere einem saugfähigen Material, wobei das Material zur Aufnahme von Urinresten, insbesondere Urintropfen, vorgesehen ist und wobei ein Wegspülen des Materials in der Toilette, insbesondere dem Urinal (10), vorgesehen ist, **dadurch gekennzeichnet, dass** das Material Polyvinylalkohol (PVA) und vollständig wasserlöslich ist.

2. Hygieneprodukt nach Anspruch 1, **dadurch gekennzeichnet, dass** das Material in kurzer Zeit wasserlöslich ist, vorzugsweise in weniger als fünf Minuten, weiter vorzugsweise in weniger als einer Minute, weiter vorzugsweise weniger als 30 Sekunden.

3. Hygieneprodukt nach einem der vorhergehenden Ansprüche, **dadurch gekennzeichnet, dass** das Material zumindest durch das Wasser (15) der Spülung der Toilette, insbesondere dem Urinal (10), auflösbar und/oder wegspülbar ist, wobei vorzugsweise das Wasser (15) mit dem aufgelösten Material abläuft, vorzugsweise durch den Abfluss der Toilette.

4. Hygieneprodukt nach einem der vorhergehenden Ansprüche, **dadurch gekennzeichnet, dass** das Material während weniger Spülvorgänge wasserlöslich und/oder wegspülbar ist, vorzugsweise zumindest im Wesentlichen innerhalb von zwei Spülvorgängen, weiter vorzugsweise innerhalb eines Spülvorgangs.

5. Hygieneprodukt nach einem der vorhergehenden Ansprüche, **dadurch gekennzeichnet, dass** das Material ein textiles Material ist, wobei vorzugsweise das Material ein nicht-verwobenes Material ist, insbesondere ein Vlies beziehungsweise Vliesstoff, und/oder ein verwobenes Material, insbesondere ein Gewebe, wobei vorzugsweise Vlies mit einem Flächengewicht von zwischen 40 und 60 Gramm pro Quadratmeter, vorzugsweise wenigstens 30 bis 35 Gramm pro Quadratmeter, besonders bevorzugt etwa 50 Gramm pro Quadratmeter.

6. Hygieneprodukt nach einem der vorhergehenden Ansprüche, **dadurch gekennzeichnet, dass** das Material ein biologisch kompatibles Material ist, vorzugsweise ein biologisch abbaubares Material, vorzugsweise kompostierbares Material.

7. Hygieneprodukt nach einem der vorhergehenden Ansprüche, **dadurch gekennzeichnet, dass** das Material ein Kunststoff ist, insbesondere ein Bio-Kunststoff.

8. Hygieneprodukt nach einem der vorhergehenden Ansprüche, **dadurch gekennzeichnet, dass** das Material zur portionsweisen Verwendung vorgesehen ist, wobei vorzugsweise Portionen des Materials vordefiniert sind oder das Material zur Benutzung in Portionen zerteilbar ist.

9. Spender zur Abgabe eines Hygieneprodukts (12), insbesondere für Männer, nach einem der vorhergehenden Ansprüche, zur Anordnung in der Nähe einer Toilette, insbesondere eines Urinals (10), wobei eine Abgabe des Materials des Hygieneprodukts (12) an eine Person vorgesehen ist, **dadurch gekennzeichnet, dass** das Material portionsweise abgebbar ist.

10. Spender nach Anspruch 9, **dadurch gekennzeichnet, dass** das Hygieneprodukt (12) beziehungsweise das Material durch eine Person entnehmbar ist.

11. Spender nach Anspruch 9 oder 10, **dadurch gekennzeichnet, dass** bei der Entnahme des Hygieneprodukts (12) beziehungsweise des Materials eine Portionierung erfolgt, vorzugsweise durch die Person.

12. Spender nach einem der Ansprüche 9 bis 11, **dadurch gekennzeichnet, dass** eine automatische Abgabe und/oder eine automatische Portionierung des Hygieneprodukts (12) beziehungsweise des Materials vorgesehen ist, wobei vorzugsweise eine motorische Abgabe und/oder Portionierung vorgesehen ist, und/oder dass eine manuelle Entnahme von Hygieneprodukten aus dem Spender durch eine Person vorgesehen ist, vorzugsweise eine vollständig manuelle Entnahme.

## Claims

1. Hygiene product, in particular for men, for cleaning the intimate area after urinating at a toilet, in particular a urinal (10), formed from a material for absorbing liquid, in particular an absorbent material, the material being intended for absorbing urine residues, in particular urine drops, and the material being intended to be flushed away in the toilet, in particular the urinal (10), **characterised in that** the material is polyvinyl alcohol (PVA) and is completely water-soluble.

2. Hygiene product according to claim 1, **characterised in that** the material is water-soluble in a short time, preferably in less than five minutes, more preferably in less than one minute, more preferably less than 30 seconds.

3. Hygiene product according to one of the preceding claims, **characterised in that** the material can be dissolved and/or flushed away at least by the water (15) of the flush of the toilet, in particular the urinal (10), preferably the water (15) drains away with the dissolved material, preferably through the drain of the toilet.

4. Hygiene product according to one of the preceding claims, **characterised in that** the material is water-soluble and/or can be flushed away during a few flushing operations, preferably at least essentially within two flushing operations, further preferably within one flushing operation.

5. Hygiene product according to one of the preceding claims, **characterised in that** the material is a textile material, preferably the material being a non-woven material, in particular a non-woven or non-woven fabric, and/or a woven material, in particular a woven fabric, preferably non-woven with a basis weight of between 40 and 60 grams per square metre, preferably at least 30 to 35 grams per square metre, particularly preferably about 50 grams per square metre.

6. Hygiene product according to any of the preceding claims, **characterised in that** the material is a biocompatible material, preferably a biodegradable material, preferably compostable material.

7. Hygiene product according to one of the preceding claims, **characterised in that** the material is a plastic, in particular a bioplastic.

8. Hygiene product according to one of the preceding claims, **characterised in that** the material is intended for use in portions, wherein preferably portions of the material are predefined or the material can be divided into portions for use.

9. Dispenser for dispensing a hygiene product (12), in particular for men, according to one of the preceding claims, for arrangement in the vicinity of a toilet, in particular a urinal (10), wherein a dispensing of the material of the hygiene product (12) to a person is provided, **characterised in that** the material can be dispensed in portions.

10. Dispenser according to claim 9, **characterised in that** the hygiene product (12) or the material can be taken out by a person.

11. Dispenser according to claim 9 or 10, **characterised in that** when the hygiene product (12) or the material is taken out, it is portioned, preferably by the person.

12. Dispenser according to one of claims 9 to 11, **characterised in that** automatic dispensing and/or automatic portioning of the hygiene product (12) or the material is provided, preferably motor-driven dispensing and/or portioning is provided, and/or **in that** manual removal of hygiene products from the dispenser by a person is provided, preferably completely manual taking out.

## Revendications

1. Produit d'hygiène, en particulier pour hommes, destiné au nettoyage l'intimité après avoir uriné dans une toilette, en particulier dans un urinoir (10), constitué d'un matériau destiné à absorber des liquides, en particulier d'un matériau absorbant, le matériau étant destiné à absorber les résidus d'urine, en particulier les gouttes d'urine, et le matériau pouvant être évacué dans la toilette, en particulier dans l'urinoir (10), **caractérisé en ce que** le matériau est de l'alcool polyvinylique (PVA) et est entièrement soluble dans l'eau.

2. Produit d'hygiène selon la revendication 1, **caractérisé en ce que** le matériau est soluble dans l'eau en peu de temps, de préférence en moins de cinq minutes, de préférence en moins d'une minute, de préférence en moins de 30 secondes.

3. Produit d'hygiène selon l'une des revendications précédentes, **caractérisé en ce que** le matériau est soluble et/ou rinçable au moins par l'eau (15) de la chasse d'eau de la toilette, en particulier de l'urinoir (10), l'eau (15) s'écoulant de préférence avec le matériau dissous, de préférence par l'évacuation de la toilette.

4. Produit d'hygiène selon l'une des revendications précédentes, **caractérisé en ce que** le matériau est soluble dans l'eau et/ou peut être évacué par rinçage en moins de cycles de rinçage, de préférence au moins essentiellement en deux cycles de rinçage, de préférence en un cycle de rinçage.

5. Produit d'hygiène selon l'une des revendications précédentes, **caractérisé en ce que** le matériau est un matériau textile, le matériau étant de préférence un matériau non tissé, en particulier un non-tissé ou un tissu non tissé, et/ou un matériau tissé, en particulier un tissu, de préférence un non-tissé d'un poids sur surface compris entre 40 et 60 grammes par mètre carré, de préférence d'au moins 30 à 35 grammes par mètre carré, de préférence d'environ 50 grammes par mètre carré.

6. Produit d'hygiène selon l'une des revendications précédentes, **caractérisé en ce que** le matériau est un matériau biocompatible, de préférence un matériau biodégradable, de préférence un matériau compostable.

7. Produit d'hygiène selon l'une des revendications précédentes, **caractérisé en ce que** le matériau est une matière plastique, en particulier une bioplastique.

8. Produit d'hygiène selon l'une des revendications précédentes, **caractérisé en ce que** le matériau est prévu pour une utilisation par portions, des portions du matériau étant de préférence prédéfinies ou le matériau pouvant être divisé en portions pour l'utilisation.

9. Distributeur pour distribuer un produit d'hygiène (12), en particulier pour hommes, selon l'une des revendications précédentes, destiné à être disposé à proximité d'une toilette, en particulier d'un urinoir (10), une distribution du matériau du produit d'hygiène (12) à une personne étant prévue, **caractérisé en ce que** le matériau peut être distribué en portions.

10. Distributeur selon la revendication 9, **caractérisé en ce que** le produit d'hygiène (12) ou le matériau peut être prélevé par une personne.

11. Distributeur selon la revendication 9 ou 10, **caractérisé en ce que** lors du prélèvement du produit d'hygiène (12) ou du matériau, un portionnement est effectué, de préférence par la personne.

12. Distributeur selon l'une des revendications 9 à 11, **caractérisé en ce qu'**une distribution automatique et/ou un portionnement automatique du produit d'hygiène (12) ou du matériau est prévu, une distribution et/ou un portionnement motorisé étant de préférence prévu, et/ou **en ce qu'**un retrait manuel des produits d'hygiène du distributeur par une personne est prévu, de préférence un retrait entièrement manuel.
